# FASCICULE DE BREVET EUROPEEN

(11) **EP 0 493 461 B1**
(45) Date de publication et mention de la délivrance du brevet: **06.04.1994**
(21) Numéro de dépôt: 90914201.0
(22) Date de dépôt: 25.09.1990
(51) Int. Cl.: A23P 1/08, A23G 3/26, A23G 3/20, A61K 9/28

(54) **MACHINE ET PROCEDE POUR LE TRAITEMENT EN CONTINU DE SURFACE D'ARTICLES DE GROSSEUR REDUITE**
VORRICHTUNG UND VERFAHREN ZUR KONTINUIERLICHEN OBERFLÄCHENBEHANDLUNG VON ARTIKELN MIT GERINGER GRÖSSE
MACHINE AND METHOD FOR THE CONTINUOUS TREATMENT OF SURFACES OF ARTICLES OF REDUCED THICKNESS

(30) Priorité: 27.09.1989 FR 8912866
(43) Date de publication de la demande: 08.07.1992
(73) Titulaire: GONTERO, Roger, La Vivette, F-13080 Luynes (FR); SERRA, Claude, F-13080 Luynes (FR)
(72) Inventeur: GONTERO, Roger, La Vivette, F-13080 Luynes (FR); SERRA, Claude, F-13080 Luynes (FR)
(74) Mandataire: Marek, Pierre, Cabinet Marek
(86) Numéro de dépôt international: FR9000684
(87) Numéro de publication internationale: WO9104679

(56) Documents cités:
- EP-A- 0 192 012
- FR-A- 2 596 956

## Description

La présente invention concerne une machine et un procédé pour le traitement en continu de surface d'articles de grosseur réduite, par exemple pour le traitement de produits fragiles de faibles dimensions.

Cette machine et ce procédé sont avantageusement applicables à l'exécution d'opérations diverses de traitement de surface de petits articles, dont une énumération non exhaustive est donnée ci-après :
- enrobage, à chaud ou froid, avec un adjuvant liquide et/ou pulvérulent, de produits tels que bonbons et autres produits de confiserie, biscuits, cacahuètes, fruits secs, semences, céréales éclatées, produits gélifiés, produits extrudés, etc. ;
- "givrage" de bonbons et autres produits de confiserie (rayage des produits enrobés d'une solution à base de sucre, pour éviter qu'ils s'amalgament) ;
- "huilage" de cacahuètes et produits gélifiés (application d'une mince couche d'un adjuvant huileux, pour obtenir un aspect de finition) ;
- "gommage" (dépôt d'une mince couche de gomme destinée à retenir un produit pulvérulent autour d'un noyau solide ou compact, par exemple pour retenir le sel sur certains produits, le sucre sur les pâtes de fruit, les dattes, etc. ; ou pour maintenir la compacité de produits friables, par exemple dépôt d'une mince couche de gomme autour du noyau constitué par le principe actif des cachets pharmaceutiques) ;
- mélange de produits, à chaud ou à froid, par exemple mélange de fruits secs, de légumes rapés, etc. ;
- pelliculage ou dépelliculage de graines, semences, cacahuètes, etc.
- "démottage" de produits (par exemple séparation de fruits confits ou autres produits agglutinés) ;
- tamisage et calibrage ;
- brossage ou lustrage.

On connaît (EP-A-0.192.012) une machine pour enrober uniformément, en continu, des produits de confiserie comportant un noyau solide, ou gélatineux, ou pâteux, enrobé dans des produits Liquides et/ou pulvérulents, du type comportant une goulotte fixe, sensiblement horizontale, une vis sans fin qui est entraînée en rotation dans ladite goulotte et des moyens pour introduire dans ladite goulotte lesdits noyaux et lesdits produits liquides et/ou pulvérulents, cette machine comportant encore une ou plusieurs brosses hélicoïdales qui sont composées de poils implantés radialement en hélice sur un ou deux arbres d'entraînement qui sont entraînés en rotation dans ladite goulotte à une vitesse inférieure à 100 tours/minute et ladite goulotte a la forme d'une auge qui est ouverte vers le haut, qui a une hauteur supérieure au diamètre desdites brosses et qui a un fond cylindrique qui enveloppe lesdites brosses dans la partie située au-dessous desdits arbres d'entraînement.

Les performances d'une telle machine sont médiocres. En effet, les produits sont poussés dans un seul sens par la brosse hélicoïdale et s'il s'agit de produits ne présentant pas une conformation arrondie (cachets, rondelles de pommes de terre frites, etc.), ces produits glissent sur la paroi de la goulotte au lieu de tourner sur eux-mêmes d'où :
- un mauvais enrobage des produits si la machine est utilisée pour cette fonction ;
- une séparation insuffisante des unités du produit, avec un effet de colmatage puis de bourrage qui bloque la machine, lorsqu'on veut mettre en oeuvre ladite machine pour exécuter des opérations de "givrage" ;
- un brossage médiocre des produits si l'on utilise la machine à cette fin. Pour obtenir un résultat convenable, il faudrait allonger la goulotte et la brosse helicoïdale, et augmenter le temps de passage des produits dans ladite goulotte.

D'autre part, lorsqu'on utilise cette machine pour traiter des produits visqueux, collants ou gluants ou tout autre produit, avec un adjuvant liquide ou pulvérulent, il se produit un encrassement rapide et massif de l'âme des brosses, sans aucune possibilité de nettoyage de celles-ci pendant le fonctionnement de ladite machine.

On connaît aussi (FR-A-2.596.956) une machine pour fabriquer des produits enrobés, comportant : - un cylindre tournant à axe sensiblement horizontal dont l'extrémité aval est ouverte ; - une brosse hélicoïdale à poils souples disposées à l'intérieur dudit cylindre, à l'extrémité amont de celui-ci, et qui est solidaire en rotation dudit cylindre; - et un convoyeur composé d'une deuxième brosse hélicoïdale à poils souples, qui est entraînée en rotation à l'intérieur d'un tube fixe qui est coaxial audit cylindre tournant et qui pénétre légèrement à l'intérieur de l'extrémité amont de celui-ci.

Cette machine est affectée d'un premier inconvénient important constitué par le fait que le cylindre rotatif et la brosse hélicoïdale montée fixement à l'intérieur de celui-ci tournent en même temps, et dans le même sens, de sorte que les unités ou particules de produits ne sont pas brossées et agitées dans tous les sens, mais poussées en direction de la sortie en glissant sur la paroi interne dudit cylindre, ce qui outre une médiocre action de brassage et d'enrobage, engendre un effet de masse par accumulation des produits qui, lorsqu'ils sont fragiles (tranches de pommes de terre frites ou autres), peuvent se briser et s'effriter. Ce phénomène nuisible est accentué par la présence, à l'intérieur du cylindre, des entretoises radiales qui relient rigidement ledit cylindre et l'arbre axial de la brosse hélicoïdale.

En outre, le nettoyage d'une telle machine dont les surfaces internes se recouvrent rapidement de dépôts résultant de l'utilisation de produits visqueux ou sirupeux, est très difficile à réaliser.

Un objet de la présente invention est donc de remédier aux inconvénients et insuffisances des machines d'enrobage du genre comportant une brosse hélicoïdale logée dans un conduit rectiligne disposé horizontalement ou ssensiblement horizontalement.

Selon l'invention, ce but est atteint grâce à un procédé et à une machine suivant lesquels le produit à traiter est introduit dans un cylindre tournant disposé horizontalement ou sensiblement horizontalement et dans lequel est montée au moins une brosse hélicoïdale rotative tournant dans un sens opposé au sens de rotation dudit cylindre, ladite brosse se trouvant en contact, par l'intermédiaire d'au moins une génératrice, avec au moins une génératrice interne de la partie inférieure de ce dernier.

Selon une autre disposition caractéristique, la brosse hélicoïdale a un diamètre inférieur au diamètre intérieur du cylindre et elle est placée en contact tangentiel avec la paroi interne de celui-ci.

Selon une autre disposition caractéristique, une plaque fixe est placée à l'intérieur du cylindre, face à la brosse hélicoïdale et parallèlement ou sensiblement parallèlement à cette dernière, cette plaque étant séparée de ladite brosse et comportant avantageusement une partie inférieure verticale ou sensiblement verticale et une partie supérieure disposée obliquement et orientée en direction de la partie supérieure de la surface latérale interne du cylindre.

L'invention permet d'obtenir plusieurs résultats avantageux. Elle autorise une grande variété d'opérations de traitement de surface sur des produits de dimensions réduites et de nature diverse, notamment sur des produits fragiles s'effritant facilement. Elle donne des résultats meilleurs que ceux qu'il est permis d'obtenir actuellement par la mise en oeuvre des matériels connus susmentionnés, en raison d'un meilleur brassage des produits. Elle réduit la rapidité et l'importance de l'encrassement de l'âme de la brosse hélicoïdale. Elle permet un nettoyage automatique de la paroi interne du cylindre Elle rend possible l'exécution d'une grande variété d'opérations de traitement de surface de produits divers se présentant sous forme d'unités ou de parcelles, introduits en continu et en vrac dans la machine de traitement.

Les buts, caractéristiques et avantages ci-dessus, et d'autres encore, ressortiront mieux de la description qui suit et des dessins annexés dans lesquels:

La figure 1 est une vue de face, avec coupes partielles, de la machine de traitement de surface selon l'invention.

La figure 2 est une vue en coupe suivant la ligne 2-2 de la figure 1.

La figure 3 est une vue en coupe selon la ligne 3-3 de la figure 1.

La figure 4 est une vue en perspective, à caractère schématique et avec coupe partielle, d'une variante d'exécution de la machine de traitement de surface selon l'invention.

La figure 5 est une vue en coupe transversale de cette machine.

La figure 6 est une vue en coupe longitudinale, suivant la ligne 6-6 de la figure 5.

La figure 7 est une vue de détail montrant un exemple de réalisation de l'un des supports réglables de la brosse hélicoïdale.

La figure 8 est une vue de détail montrant un exemple d'exécution de l'un des supports réglables de la plaque fixe.

La figure 9 est une vue de détail illustrant l'un des supports réglables du cylindre tournant.

On se réfère auxdits dessins pour décrire des exemples intéressants, bien que nullement limitatifs, d'exécution de la machine et de mise en oeuvre du procédé selon l'invention.

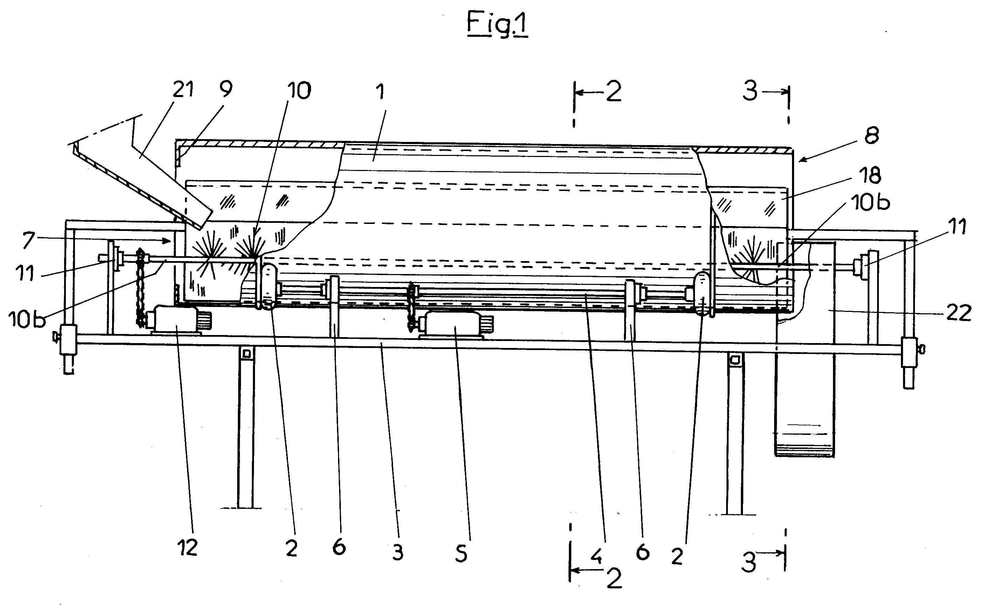

Selon le mode de réalisation illustré aux figures 1 à 3, cette machine comporte un cylindre tournant 1 disposé horizontalement ou avec une inclination réduite. Ce cylindre est supporté par au moins deux paires de galets 2 à axe horizontal ou sensiblement horizontal.

Les galets 2 disposés sur l'un des côtés du cylindre 1 sont calés sur un arbre moteur 4 entrainé en rotation par un moteur électrique 5 assurant, par l'intermédiaire dudit arbre et desdits galets, l'entraînement en rotation du cylindre 1 à vitesse lente et réglable, par exemple comprise entre 1 tour/minute et 40 tours/minute, suivant la nature des produits traités. La liaison entre le moteur 5 et l'arbre moteur 4 peut être assurée par une transmission classique à chaîne et pignons, comprenant, en outre, un dispositif de réglage de tension de chaîne.

Les paliers de l'arbre 4 ou les galets 2 sont portés par des supports verticaux 6 solidaires du bâti 3 de la machine.

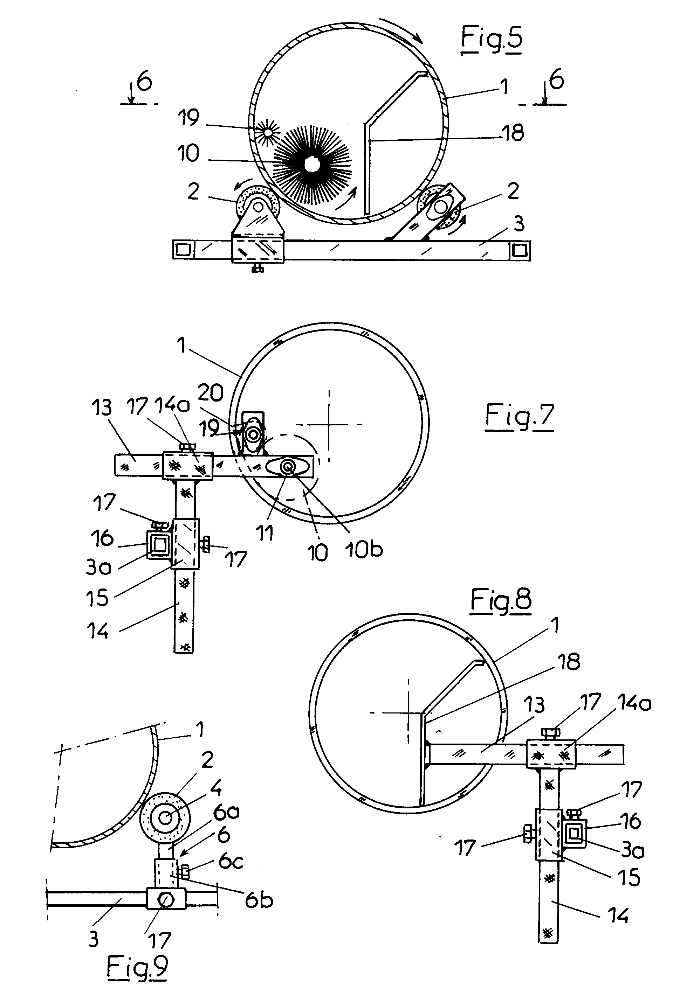

Le cylindre 1 peut être avantageusement monté avec une inclinaison Limitée et réglable. Pour celà, les supports 6 portant les galets 2 ou les paliers de l'arbre 4, sont agencés, de manière connue en soi, pour permettre un réglage de leur hauteur. Comme illustré à la figure 9, les supports 6 peuvent, par exemple, être exécutés en deux parties tubulaires 6a, 6b assemblées de manière télescopique et équipés d'une vis de blocage 6c permettant l'assemblage rigide de ces deux parties dans la position désirée. De la sorte, en inclinant le cylindre 1 de façon que son extrémité postérieure soit surélevée par rapport à son extrémité antérieure, on ralentit la vitesse de déplacement des produits dans le cylindre et on augmente la durée du traitement sans nécessité d'accroître la longueur dudit cylindre.

Les extrémités du cylindre 1 sont ouvertes pour constituer, respectivement, une ouverture d'entrée 7 et une ouverture de sortie 8. L'extrémité aval délimitant la sortie 8 est totalement ouverte, tandis que l'extrémité amont est partiellement fermée par un rebord 9 en forme de couronne circulaire délimitant l'entrée 7 et destiné à retenir les unités ou parcelles du produit traité qui pourraient rouler ou glisser en direction de ladite entrée, lors de l'introduction dudit produit dans le cylindre.

Dans le cylindre 1, est logée, parallèlement à l'axe dudit cylindre, au moins une brosse hélicoïdale 10 dont la longueur peut être à peine inférieure à celle de celui-ci. Cette brosse helicoïdale est constituée, de manière connue en soi, de poils souples 10a, par exemple exécutés en matière plastique alimentaire ou en acier inoxydable et implantés hélicoïdalement autour d'un arbre axial 10b avantageusement réalisé en acier inoxydable ou en matière plastique rigide telle que polyamide.

La brosse hélicoïdale 10 peut être à simple ou double filet et elle est montée tournante dans un sens (Flèche F1) inverse au sens de rotation (F2) du cylindre 1. Plus précisément, le cylindre 1 est entraîné en rotation dans un sens opposé au sens de rotation actif de la brosse héicoïdale, c'est-à-dire au sens assurant l'entraînement du produit en direction de l'extrémité aval dudit cylindre. Les extrémités de l'arbre 10b sont montées dans des paliers 11 portés par des éléments verticaux du bâti 3 de la machine et l'entraînement en rotation de la brosse hélicoïdale à vitesse lente et réglable, par exemple comprise entre 30 tours/minute et 100 tours/minute, est assuré par un moteur électrique 12 également installé sur ledit bâti et par l'intermédiaire d'une transmission, par exemple à chaîne et pignons.

La brosse hélicoïdale 10 se trouve en contact, par l'intermédiaire d'au moins une génératrice, avec au moins une génératrice G de la surface interne de la partie inférieure du cylindre 1.

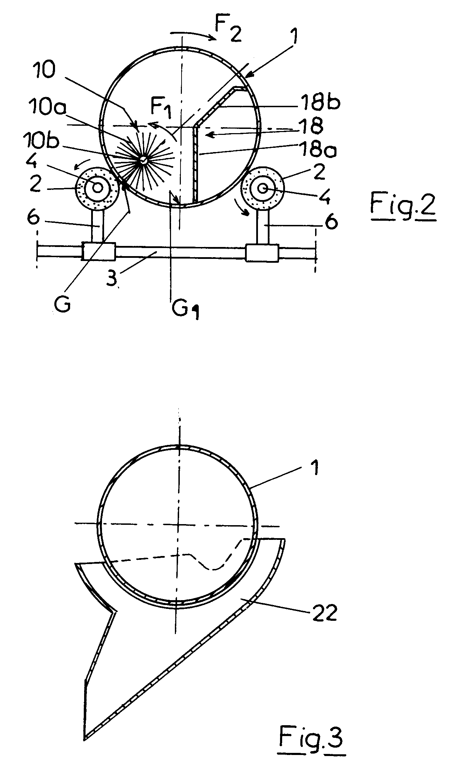

De manière préférée et avantageuse, la brosse hélicoïdale 10 a un diamètre nominal inférieur au diamètre intérieur du cylindre 1 et elle est logée dans la demi-partie inférieure de ce dernier, de manière à être placée en contact tangentiel avec au moins une génératrice G de la surface interne de la partie inférieure dudit cylindre distante de la génératrice la plus basse G1 de ce dernier (figure 2).

La brosse hélicoïdale 10 peut, par exemple, avoir un diamètre nominal légèrement inférieur au rayon du cylindre 1.

La position de la brosse hélicoïdale 10 par rapport à la génératrice interne la plus basse G1 du cylindre 1, est réglable. Dans ce but, les éléments du bâti qui soutiennent l'arbre axial 10b de la brosse hélicoïdale 10 peuvent être réalisés en plusieurs parties assemblées de façon réglable, de manière connue en soi, par exemple au moyen de manchons ou colliers et de vis de blocage. Un exemple d'exécution de tels supports réglables est représenté succintement à la figure 7 où l'on voit l'un des paliers 11 de la brosse hélicoïdales 10 porté par un bras horizontal 13 monté avec une aptitude de coulissement dans un manchon à axe horizontal 14a équipant le sommet d'un montant 14 également monté avec une latitude de glissement dans un manchon à axe vertical 15 rigidement solidaire d'un collier 16 qui peut être déplacé le long d'un élément longitudinal 3a du bâti 3 de la machine. Des vis 17 se vissant dans les manchons 14a, 15 et dans le collier 16, permettent d'immobiliser les différents éléments des supports réglables, dans la position désirée.

Dans le cylindre 1, est encore disposée longitudinalement et fixement une plaque 18 dont la longueur correspond sensiblement à la longueur dudit cylindre ; la longueur de la plaque 18 étant, par exemple, à peine inférieure à celle de ce dernier.

Cette plaque 18 qui reste fixe lors de la rotation du cylindre, est disposée face à la brosse hélicoïdale 10, à faible distance de cette dernière et parallèlement ou sensiblement parallèlement à ladite brosse. Elle comporte une partie inférieure verticale ou sensiblement verticale 18a placée face à la brosse hélicoïdale 10, et une partie supérieure 18b disposée obliquement et s'élevant dans la demi-partie supérieure du cylindre en direction de la surface Latérale interne de ce dernier.

Un espace est ménagé entre le bord inférieur de la plaque fixe 18 et la surface latérale du cylindre 1, afin que les quelques parcelles ou unités du produit traité qui pourraient retomber à l'arrière de ladite plaque puissent être ramenées en direction de la brosse hélicoïdale 10.

La position de la plaque 18 peut être réglée par des moyens analogues à ceux qui permettent le réglage de la brosse hélicoïdale 10. La figure 8 illustre un exemple de réalisation d'un support réglable dont les différents éléments comparables aux éléments du support de brosse réglable représenté à la figure 7, sont désignés par les mêmes références.

Les dispositifs de réglage de la position de la brosse hélicoïdale et de la plaque 18 permettent également de placer ladite brosse et ladite plaque dans une position inclinée correspondant à l'inclinaison du cylindre 1 lorsque celui-ci est placé dans une position inclinée en vue de l'exécution de certains traitements.

Suivant un mode d'exécution non représenté sur les dessins, il est possible de monter deux brosses hélicoïdales à l'intérieur du cylindre 1, la deuxième brosse hélicoïdale étant également placée en contact tangentiel avec la surface interne de la paroi latérale dudit cylindre. Dans ce cas, les deux brosses sont disposées parallèlement de façon que leurs filets s'interpénètrent. Elles peuvent avoir des diamètres égaux ou inégaux. En outre, ces deux brosses peuvent tourner à la même vitesse ou à der vitesses différentes.

Elles sont entrainées en rotation, en sens contraire, par un moteur électrique commun ou, chacune, individuellement, par son propre moteur d'entraînement, au moyen de tout système de transmission approprié.

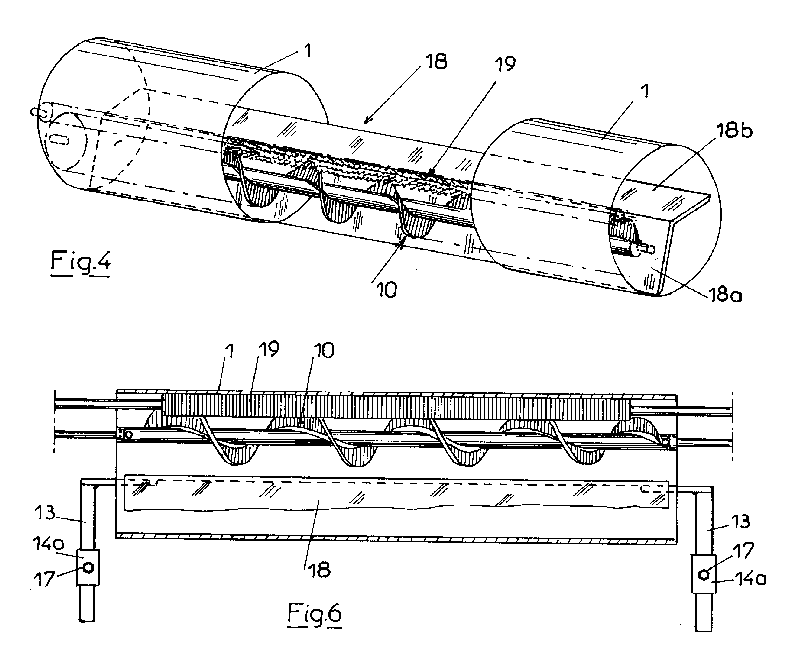

Les figures 4, 5 et 6 illustrent un mode d'exécution comparable à celui qui est représenté aux figures 1 à 3 et qui diffère de celui-ci principalement par le fait qu'un rouleau nettoyeur 19 constitué par une brosse cylindrique, est monté parallèlement à l'axe du cylindre 1 et à la brosse hélicoïdale 10, de préférence à proximité et au-dessus de cette dernière. Ce rouleau nettoyeur est monté en contact avec la surface interne de la paroi latérale du cylindre 1 et sa fonction est de maintenir cette surface parfaitement lisse en la débarrassant, en permanence, des particules qui peuvent y adhérer en cours de travail.

Le rouleau nettoyeur 19 peut être porté par des paliers 20 fixés sur les bras 13 des supports réglables de la brosse hélicoïdale 10, de mânière à permettre un réglage de la position dudit rouleau, en même temps que celle de ladite brosse.

On décrit ci-après la mise en oeuvre du procédé et le fonctionnement de la machine selon l'invention.

Le produit solide à traiter qui se présente sous forme d'unités (dattes, cacahuètes, etc.) ou de petites parcelles (fruits secs, céréales éclatées, etc.), et, suivant la nature du traitement, le produit pulvérulent (sucre, sel, etc.) ou liquide (agent de protection, colorant, etc.) de traitement, sont introduits en continu dans le cylindre 1, en amont de ce dernier. Ce ou ces produits sont, par exemple, déversés dans le cylindre 1 au moyen d'une goulotte 21 traversant l'ouverture d'entrée 7 dudit cylindre.

Les unités ou parcelles de produit et, selon la nature du traitement, la poudre et/ou le liquide de traitement tombant dans le fond du cylindre 1, sont entraînées en rotation par celui-ci et poussées en direction de l'extrémité aval dudit cylindre, par l'action de la vis hélicoïdale 10 mise en rotation dans un sens permettant cette progression et entre les spires de laquelle vient se loger le produit. Les poils constituant l'hélice de la brosse tournante, assurent également une action de brassage du produit lors de son avancement dans le cylindre. En outre, la brosse tend à repousser le produit en direction de la paroi de la demi-partie du cylindre opposée à la demi-partie de ce dernier dans laquelle elle est installée, en considérant le plan vertical médian dudit cylindre. La mise en rotation de celui-ci, dans le sens inverse au sens de rotation actif de la brosse hélicoïdale 10 a pour effet de ramener le produit entre les spires de cette dernière. Les mouvements conjugués de la brosse hélicoïdale et du cylindre ont pour effet de retourner le produit dans tous les sens, tout au long de sa progression dans le cylindre. La plaque 18 limite le déplacement latéral du produit que la brosse hélicoïdale tend à faire remonter vers le haut.

A sa sortie du cylindre, le produit traité se déverse automatiquement dans un bac ou dans une trémie 22 à partir de laquelle il peut être retiré ou dirigé vers un autre poste de traitement, par exemple au moyen d'un convoyeur.

## Revendications

1. Machine pour le traitement en continu de surface d'articles de grosseur réduite, par exemple pour le traitement de surface de produits fragiles de faibles dimensions, comprenant au moins une vis sans fin constituée par une brosse hélicoïdale (10) formée d'un arbre axial (10b) sur lequel sont implantés des poils radiaux (10a) répartis en hélice autour dudit arbre, caractérisée en ce que cette brosse hélicoïdale (10) est montée avec une aptitude de rotation dans un cylindre (1) tournant dans un sens inverse au sens de rotation actif de ladite brosse hélicoïdale, laquelle se trouve en contact, par l'intermédiaire d'au moins une génératrice, avec au moins une génératrice interne (6) de la partie inférieure dudit cylindre.

2. Machine selon la revendication 1, caractérisée en ce que la brosse hélicoïdale (10) a un diamètre nominal inférieur au diamètre intérieur du cylindre (1) et elle est placée en contact tangentiel avec la paroi interne de celui-ci.

3. Machine selon la revendication 1, caractérisée en ce que la brosse hélicoïdale (10) est logée dans la demi-partie inférieure du cylindre (1), son point de contact avec la paroi interne dudit cylindre étant distant de la génératrice la plus basse (G1) de ce dernier.

4. Machine suivant l'une quelconque des revendications 1 à 3, caractérisée en ce qu'une plaque fixe (18) est placée à l'intérieur du cylindre (1) face à la brosse hélicoïdale (10) et parallèlement ou sensiblement parallèlement à cette dernière.

5. Machine selon la revendication 4, caractérisée en ce que ladite plaque fixe (18) comprend une partie verticale ou sensiblement verticale (18a) disposée en regard de la brosse hélicoïdale (10) et une partie supérieure oblique (18b), orientée en direction de la surface latérale interne du cylindre (1).

6. Machine suivant l'une quelconque des revendications 1 à 5, caractérisée en ce qu'une seconde brosse hélicoïdale tournante est montée dans le cylindre rotatif (1) parallèlement à la première (10), ces brosses tournant en sens contraire.

7. Machine selon l'une quelconque des revendications 1 à 6, caractérisée en ce qu'elle comporte des moyens (2, 5) permettant l'entraînement en rotation du cylindre à vitesse lente, par exemple à une vitesse comprise entre 1 tour/minute et 40 tours/minute.

8. Machine suivant l'une quelconque des revendications 1 à 7, caractérisée en ce qu'elle comporte des moyens (12) permettant l'entraînement en rotation de la brosse helicoïdale (10) ou des brosses hélicoïdales, à vitesse(s) lente(s), par exemple à une vitesse comprise entre 30 tours/minute et 100 tours/minute.

9. Machine selon l'une quelconque des revendications 1 à 6, caractérisée en ce qu'elle comporte un rouleau nettoyeur (19) monté en contact avec la surface interne de la paroi latérale du cylindre (1).

10. Machine suivant l'une quelconque des revendications 1 à 6, ou selon la revendication 9, caractérisée en ce que la ou les brosses hélicoïdales (10) et/ou le rouleau nettoyeur (19) sont portés par des supports réglables (13-14-15-16) permettant un réglage de leur position.

11. Machine selon la revendication 4, caractérisée en ce que la plaque fixe (18) est portée par des supports réglables (13-14-15-16) permettant un réglage de sa position.

12. Machine selon l'une quelconque des revendications 1 à 11, caractérisé en ce que le cylindre tournant (1) est supporté par des supports réglables (6) permettant de l'incliner dans une position suivant lequelle son extrémité aval se trouvé surélevée par rapport à son extrémité amont.

13. Procédé pour le traitement en continu de surface d'articles de grosseur réduite, par exemple pour le traitement de surface de produits fragiles de faibles dimensions, caractérisé en ce que l'on déverse en continu le produit à traiter et, si nécessaire, le produit de traitement, dans un cylindre rotatif (1) disposé horizontalement ou sensiblement horizontalement, que l'on fait tourner à vitesse lente et dans lequel on fait tourner, également à vitesse réduite et en sens opposé, une brosse hélicoïdale (10) disposée parallèlement audit cylindre et que l'on met en contact par l'intermédiaire d'au moins l'une de ses génératrices, avec au moins une génératrice interne de la partie inférieure de ce dernier.

14. Procédé selon la revendication 13, caractérisé en ce que l'on met la brosse hélicoïdale (10) au contact d'une génératrice interne (6) du cylindre (1) distante de la génératrice la plus basse (G1) de ce dernier.

15. Procédé suivant l'une des revendications 13 ou 14, caractérisé en ce que l'on fait tourner, en sens contraire et à vitesse réduite, deux brosses hélicoïdales disposées parallèlement, la seconde brosse tournant dans le même sens que le cylindre (1) et se trouvant également disposée en contact tangentiel avec celui-ci.

16. Procédé selon l'une quelconque des revendications 13 à 15, caractérisé en ce que l'on fait tourner le cylindre (1) à une vitesse comprise entre 1 tour/minute et 40 tours/minute.

17. Procédé suivant l'une quelconque des revendications 13 à 16, caractérisé en ce que l'on fait tourner la brosse hélicoïdale (10) ou les brosses hélicoïdales à des vitesses comprises entre 30 tours/minute et 100 tours/minute.

18. Procédé selon l'une quelconque des revendications 13 à 17, caractérisé en ce que l'on place le cylindre (1) et ses organes internes (10,18) dans une position inclinée suivant laquelle l'extrémité aval dudit cylindre (1) est surelevée par rapport à son extrémité amont, afin de ralentir la vitesse de déplacement des produits traités et d'augmenter la durée du traitement.

## Patentansprüche

1. Maschine zur Endlosbearbeitung von Oberflächen von Gegenständen geringen Ausmaßes, beispielsweise für die Oberflächenbearbeitung von kleinen zerbrechlichen Produkten, umfassend wenigstens eine Schnecke, bestehend aus einer schraubenförmigen Bürste (10), die aus einer axialen Welle (10b) gebildet wird, auf der radial angeordnete Bürstenhaare (10a) angebracht und schraubenförmig um die Welle angeordnet sind, dadurch gekennzeichnet, daß diese schraubenförmige Bürste (10) drehbar in einem Zylinder (1) angebracht ist, der eine Drehung in der Gegenrichtung der aktiven Drehung der schraubenförmigen Bürste ausführt, die sich über mindestens eine Mantellinie mit mindestens einer inneren Mantellinie (G) des unteren Teils des Zylinders in Kontakt befindet.

2. Maschine nach Anspruch 1, dadurch gekennzeichnet, daß die schraubenförmige Bürste (10) einen Nenndurchmesser aufweist, der kleiner ist als der Innendurchmesser des Zylinders (1), und daß sie in Tangentialkontakt mit dessen Innenwand positioniert ist.

3. Maschine nach Anspruch 1, dadurch gekennzeichnet, daß die schraubenförmige Bürste (10) in der unteren Hälfte des Zylinders (1) gelagert ist, wobei ihr Kontaktpunkt mit der Innenwand des Zylinders von der tiefsten Mantellinie (G1) des Zylinders entfernt ist.

4. Maschine nach einem der Ansprüche 1 bis 3, dadurch gekennzeichnet, daß eine feststehende Platte (18) im Inneren des Zylinders (1) gegenüber der schraubenförmigen Bürste (10) und parallel oder im wesentlichen parallel zu dieser positioniert ist.

5. Maschine nach Anspruch 4, dadurch gekennzeichnet, daß die feststehende Platte (18) einen gegenüber der schraubenförmigen Bürste (10) angeordneten vertikalen oder im wesentlichen vertikalen Teil (18a) sowie einen in Richtung der Innenseitenfläche des Zylinders (1) ausgerichteten oberen schrägen Teil umfaßt.

6. Maschine nach einem der Ansprüche 1 bis 5, dadurch gekennzeichnet, daß eine zweite sich drehende schraubenförmige Bürste im Drehzylinder (1) parallel zur ersten Bürste (10) angebracht ist, wobei sich diese Bürsten in entgegengesetzten Richtungen drehen.

7. Maschine nach einem der Ansprüche 1 bis 6, dadurch gekennzeichnet, daß sie Mittel (2, 5) umfaßt, die den Antrieb zur Drehung des Zylinders mit langsamer Geschwindigkeit ermöglichen, beispielsweise mit einer Geschwindigkeit zwischen einer Umdrehung/Minute und 40 Umdrehungen/Minute.

8. Maschine nach einem der Ansprüche 1 bis 7, dadurch gekennzeichnet, daß sie Mittel (12) umfaßt, die den Antrieb zur Drehung der schraubenförmigen Bürste (10) oder der schraubenförmigen Bürsten mit langsamer/langsamen Geschwindigkeit/en ermöglichen, beispielsweise mit einer Geschwindigkeit zwischen 30 Umdrehungen/Minute und 100 Umdrehungen/Minute.

9. Maschine nach einem der Ansprüche 1 bis 6, dadurch gekennzeichnet, daß sie eine Reinigungswalze (19) umfaßt, die mit der Innenoberfläche der Seitenwand des Zylinders (1) in Kontakt stehend angebracht ist.

10. Maschine nach einem der Ansprüche 1 bis 6 oder nach Anspruch 9, dadurch gekennzeichnet, daß die schraubenförmige/n Bürste/n (10) und/oder die Reinigungswalze (19) durch verstellbare Halterungen (13 - 14 - 15 - 16) gehaltert sind, die eine Verstellung ihrer Position ermöglichen.

11. Maschine nach Anspruch 4, dadurch gekennzeichnet, daß die feststehende Platte (18) durch verstellbare Halterungen (13 - 14 - 15 - 16) gehaltert ist, die eine Verstellung ihrer Position ermöglichen.

12. Maschine nach einem der Ansprüche 1 bis 11, dadurch gekennzeichnet, daß der Drehzylinder (1) durch verstellbare Halterungen (6) gehaltert ist, die es ermöglichen, ihn in eine Position zu neigen, in der sein stromabwärts gerichtetes Ende im Vergleich zu seinem stromaufwärts gerichteten Ende erhöht liegt.

13. Verfahren zur Endlosbearbeitung von Oberflächen von Gegenständen geringen Ausmaßes, beispielsweise für die Oberflächenbearbeitung von kleinen zerbrechlichen Produkten, dadurch gekennzeichnet, daß das zu bearbeitende Produkt und, falls erforderlich, das Bearbeitungsprodukt kontinuierlich in einen horizontal oder im wesentlichen horizontal angeordneten Drehzylinder (1) eingetragen werden, der mit geringer Geschwindigkeit in Drehung versetzt wird und in dem ebenfalls mit geringer Geschwindigkeit und in Gegenrichtung eine parallel zum Zylinder angeordnete, schraubenförmige Bürste in Drehung versetzt wird, die über mindestens eine ihrer Mantellinien mit mindestens einer inneren Mantellinie des unteren Teil des Zylinders in Kontakt gebracht wird.

14. Verfahren nach Anspruch 13, dadurch gekennzeichnet, daß die schraubenförmige Bürste (10) mit einer inneren Mantellinie (G) des Zylinders (1) in Kontakt gebracht wird, die von dessen unterster Mantellinie (G1) entfernt ist.

15. Verfahren nach einem der Ansprüche 13 oder 14, dadurch gekennzeichnet, daß zwei parallel angeordnete schraubenförmige Bürsten in Gegenrichtung zueinander und mit geringer Geschwindigkeit in Drehung versetzt werden, wobei sich die zweite Bürste in die gleiche Richtung dreht wie der Zylinder (1) und auch in Tangentialkontakt zu diesem stehend angeordnet ist.

16. Verfahren nach einem der Ansprüche 13 bis 15, dadurch gekennzeichnet, daß der Zylinder (1) mit einer Geschwindigkeit zwischen einer Umdrehung/Minute und 40 Umdrehungen/Minute in Umdrehung versetzt wird.

17. Verfahren nach einem der Ansprüche 13 bis 16, dadurch gekennzeichnet, daß die schraubenförmige Bürste (10) oder die schraubenförmigen Bürsten mit Geschwindigkeiten zwischen 30 Umdrehungen/Minute und 100 Umdrehungen/Minute in Umdrehung versetzt werden.

18. Verfahren nach einem der Ansprüche 13 bis 17, dadurch gekennzeichnet, daß der Zylinder (1) und seine inneren Einrichtungen (10, 18) in eine geneigte Position gebracht werden, in der das stromabwärts gerichtete Ende des Zylinders (1) im Vergleich zu dessen stromaufwärts gerichtetem Ende erhöht liegt, um damit die Fortbewegungsgeschwindigkeit der bearbeiteten Produkte zu verlangsamen und die Bearbeitungsdauer zu erhöhen.

## Claims

1. Machine for the continuous surface treatment of articles of reduced size, for example for the surface treatment of fragile products of small dimensions, comprising at least one endless screw constituted by a helicoidal brush (10) formed by an axial shaft (10b) on which are set radial bristles (10a) distributed spirally around said shaft, characterised in that said helicoidal brush (10) is mounted for rotation in a cylinder (1) which turns in an opposite direction to the direction of active rotation of said helicoidal brush, which is in contact, via at least one generating line, with at least one internal generating line (G) on the lower portion of said cylinder.

2. Machine according to claim 1, characterised in that the helicoidal brush (10) has a nominal diameter less than the internal diameter of the cylinder (1) and said brush is positioned in tangential contact with the internal wall of said cylinder.

3. Machine according to claim 1, characterised in that the helicoidal brush (10) is housed in the lower half of the cylinder (1), its point of contact with the internal wall of said cylinder being remote from the lowest generating line (G1) of said cylinder.

4. Machine according to any of claims 1 to 3, characterised in that a fixed plate (18) is positioned inside the cylinder (1), facing the helicoidal brush (10) and parallel or substantially parallel to the latter.

5. Machine according to claim 4, characterised in that said fixed plate (18) comprises a vertical or substantially vertical portion (18a) disposed facing the helicoidal brush (10) and an oblique upper portion (18b) aligned in the direction of the internal lateral surface of the cylinder (1).

6. Machine according to any of claims 1 to 5, characterised in that a second rotating helicoidal brush is mounted in the rotary cylinder (1), parallel with the first (10), and said brushes turn in opposite directions.

7. Machine according to any of claims 1 to 6, characterised in that it includes means (2, 5) enabling the cylinder to be rotated at slow speed, for example at a speed of between 1 revolution per minute and 40 revolutions per minute.

8. Machine according to any of claims 1 to 7, characterised in that it includes means (12) enabling the helicoidal brush (10) or helicoidal brushes to be rotated at slow speed(s), for example at a speed of between 30 revolutions per minute and 100 revolutions per minute.

9. Machine according to any of claims 1 to 6, characterised in that it includes a cleaning roller (19) mounted in contact with the internal surface of the lateral wall of the cylinder (1).

10. Machine according to any of claims 1 to 6, or according to claim 9, characterised in that the helicoidal brush or brushes (10) and/or the cleaning roller (19) are carried by adjustable supports (13-14-15-16) enabling their position to be adjusted.

11. Machine according to claim 4, characterised in that the fixed plate (18) is carried by adjustable supports (13-14-15-16) enabling its position to be adjusted.

12. Machine according to any of claims 1 to 11, characterised in that the rotating cylinder (1) is supported by adjustable supports (6) enabling it to be inclined in a position in which its downstream end is raised with respect to its upstream end.

13. Method for the continuous surface treatment of articles of reduced size, for example for the surface treatment of fragile products of small dimensions, characterised by continuously pouring the product to be treated and, if necessary, the treatment product, into a rotary cylinder (1) disposed horizontally or substantially horizontally, slowly rotating it, and rotating in the opposite direction, likewise at slow speed, a helicoidal brush (10) disposed parallel to said cylinder, and bringing said product into contact, via at least one of its generating lines, with at least one internal generating line of the lower portion of said cylinder.

14. Method according to claim 13, characterised by bringing the helicoidal brush (10) into contact with an internal generating line (G) of the cylinder (1) remote from the lowest generating line (G1) thereof.

15. Method according to either of claims 13 and 14, characterised by rotating two parallel helicoidal brushes in opposite directions at slow speed, the second brush turning in the same direction as the cylinder (1) and likewise being disposed in tangential contact therewith.

16. Method according to any of claims 13 to 15, characterised by rotating the cylinder (1) at a speed of between 1 revolution per minute and 40 revolutions per minute.

17. Method according to any of claims 13 to 16, characterised by rotating the helicoidal brush (10) or helicoidal brushes at speeds of between 30 revolutions per minute and 100 revolutions per minute.

18. Method according to any of claims 13 to 17, characterised by positioning the cylinder (1) and its internal elements (10, 18) in an inclined position in which the upstream end of said cylinder (1) is raised with respect to its downstream end, in order to slow the speed at which the treated products are moving and increase the duration of treatment.
